# EUROPEAN PATENT APPLICATION

(11) **EP 2 468 342 A1**
(43) Date of publication of application: **27.06.2012**
(21) Application number: 10196079.7
(22) Date of filing: 21.12.2010
(51) Int. Cl.: A61M 5/20, A61M 5/00

(54) **Front end for an auto-injector**

(71) Applicant: Sanofi-Aventis Deutschland GmbH, 65929 Frankfurt (DE)
(72) Inventor: The designation of the inventor has not yet been filed

(57) **Abstract**

The invention relates to a front end (1) for an auto-injector, the front end (1) comprising a syringe (5) with an injection needle (6) and a stopper (7), further comprising an outer sleeve (2) arranged to be attached to an auto-injector back end comprising a drive means for advancing the syringe (5) and needle (6) in a proximal direction (P) for needle insertion and for advancing the stopper (7) within the syringe (5) for injection, wherein the syringe (5) is slidably arranged within a transfer sleeve (3) which is slidably arranged in the outer sleeve (2), wherein the transfer sleeve (3) is biased in the proximal direction (P) against the syringe (5), wherein a locking mechanism is arranged for limiting a proximal extension of the transfer sleeve (3) from the outer sleeve (2) to an initial position in an initial state, wherein the locking mechanism is arranged to allow the transfer sleeve (3) to be extended into a needle safe position covering the advanced needle (6) beyond the initial position after having been translated in the distal direction (D) into a depressed position.

## Description

### Technical Field

The invention relates to front end for an auto-injector according to the preamble of claim 1.

### Background of the Invention

Administering an injection is a process which presents a number of risks and challenges for users and healthcare professionals, both mental and physical.

Injection devices (i.e. devices capable of delivering medicaments from a medication container) typically fall into two categories - manual devices and auto-injectors.

In a manual device - the user must provide the mechanical energy to drive the fluid through the needle. This is typically done by some form of button / plunger that has to be continuously pressed by the user during the injection. There are numerous disadvantages to the user from this approach. If the user stops pressing the button / plunger then the injection will also stop. This means that the user can deliver an under dose if the device is not used properly (i.e. the plunger is not fully pressed to its end position). Injection forces may be too high for the user, in particular if the patient is elderly or has dexterity problems.

The extension of the button/plunger may be too great. Thus it can be inconvenient for the user to reach a fully extended button. The combination of injection force and button extension can cause trembling / shaking of the hand which in turn increases discomfort as the inserted needle moves.

Auto-injector devices aim to make self-administration of injected therapies easier for patients. Current therapies delivered by means of self-administered injections include drugs for diabetes (both insulin and newer GLP-1 class drugs), migraine, hormone therapies, anticoagulants etc.

Auto-injectors are devices which completely or partially replace activities involved in parenteral drug delivery from standard syringes. These activities may include removal of a protective syringe cap, insertion of a needle into a patient's skin, injection of the medicament, removal of the needle, shielding of the needle and preventing reuse of the device. This overcomes many of the disadvantages of manual devices. Injection forces / button extension, hand-shaking and the likelihood of delivering an incomplete dose are reduced. Triggering may be performed by numerous means, for example a trigger button or the action of the needle reaching its injection depth. In some devices the energy to deliver the fluid is provided by a spring.

US 2002/0095120 A1 discloses an automatic injection device which automatically injects a pre-measured quantity of fluid medicine when a tension spring is released. The tension spring moves an ampoule and the injection needle from a storage position to a deployed position when it is released. The content of the ampoule is thereafter expelled by the tension spring forcing a piston forward inside the ampoule. After the fluid medicine has been injected, torsion stored in the tension spring is released and the injection needle is automatically retracted back to its original storage position.

Most conventional art auto-injectors are disposable, i.e. they are used and completely disposed of after use. For reasons of sustainability it may be desirable to re-use at least a part of the auto-injector. An auto-injector may therefore consist of a re-usable backend engine containing drive means and a front end containing the syringe with the needle which must not be re-used.

### Summary of the Invention

It is an object of the present invention to provide an improved front end for an auto-injector.

The object is achieved by a front end according to claim 1.

Preferred embodiments of the invention are given in the dependent claims.

In the context of this specification the term proximal refers to the direction pointing towards the patient during an injection while the term distal refers to the opposite direction pointing away from the patient.

According to the invention a front end for an auto-injector comprises a syringe with an injection needle and a stopper, an outer sleeve arranged to be attached to an auto-injector back end comprising a drive means for advancing the syringe and needle in a proximal direction for needle insertion and for advancing the stopper within the syringe for injecting a liquid medicament initially stored inside the syringe. The syringe is slidably arranged within a transfer sleeve which is slidably arranged in the outer sleeve. The transfer sleeve is biased in the proximal direction against the syringe. A locking mechanism is arranged for limiting a proximal extension of the transfer sleeve from the outer sleeve to an initial position in an initial state. The locking mechanism is arranged to allow the transfer sleeve to be extended into a needle safe position covering the advanced needle beyond the initial position after having been translated in the distal direction into a depressed position. The locking mechanism ensures that the front end is post injection needle safe.

In one embodiment the locking mechanism comprises a locking sleeve arranged over the transfer sleeve and coupled to the transfer sleeve for joint translation. The locking sleeve exhibits at least one fourth rib, preferably a series of fourth ribs circumferentially arranged on the locking sleeve in the manner of a shouldered castellation, wherein the fourth ribs are distally ramped. In the initial position the fourth ribs are engaged in respective first longitudinal grooves within the outer casing preventing relative rotation. On translation into the depressed position the fourth ribs are moved out of the first longitudinal grooves against respective ramp features in the outer sleeve or on the transfer sleeve in a manner to rotate the locking sleeve by a defined angle thereby aligning the fourth rib with a second longitudinal groove extending further in the proximal direction than the first longitudinal groove thus allowing translation into the depressed position. The locking mechanism according to this embodiment replicates a 'ballpoint pen' extension/retraction mechanism. The locking mechanism may also be applied in a re-usable or disposable auto-injector containing all components within one single housing.

A syringe support sleeve may be telescoped within the transfer sleeve, wherein the syringe support sleeve is arranged to hold the syringe and support it at its proximal end. Supporting the syringe at the proximal end is preferred over holding it by the finger flange since the proximal end is considerably more robust than the finger flanges and less prone to break under impact load.

The syringe support sleeve may be biased in the distal direction by a syringe spring towards an initial position. The syringe support sleeve may be arranged to be translated proximally into an insertion position for needle insertion and to be re-translated into the initial position by the syringe spring for retracting the needle from the injection site.

A syringe cover may be provided arranged to be removably positioned over a substantial length of the outer sleeve and clipped to the outer sleeve. The syringe cover may be arranged to prevent access to the internals of the front end when attached to the outer sleeve prior to an injection. After the injection the syringe cover may be re-attached to the outer sleeve for improving post injection needle safety.

The syringe cover may be arranged to engage a protective needle sheath arrangeable over the needle in a manner to remove the protective needle sheath on removal of the syringe cover. This reduces the risk of needle stick injuries since the user does not have to grip the protective needle sheath with their fingers.

The transfer sleeve may comprise at least one distal extension for engaging a sensor in the re-usable backend to indicate that the transfer sleeve is in the depressed position. Depending on the design of the back end the extension can be used to mechanically interlock an activating means such as a trigger button for preventing actuation of the activating means before the front end has been properly pushed against the injection site thus enforcing a sequence of operation with at least two steps: pushing the front end against the injection site and actuating the activating means. Needle safety prior to injection is thus improved. The extension may also be used to engage an electronic switch to interlock an electrically driven back end.

At least one internal clip may be arranged in the syringe support sleeve for engaging a finger flange of the syringe in a manner to prevent translation of the syringe in the distal direction. This keeps the syringe from falling off the distal end of the syringe support sleeve when the front end is not attached to the back end and held with the needle up. The internal clip may be distally ramped for facilitating insertion of the syringe into the syringe support sleeve during assembly.

A transfer spring may be arranged between the transfer sleeve and the syringe support sleeve for biasing them against each other. The transfer spring is arranged for advancing the transfer sleeve into the needle safe position on removal from the injection site.

The outer sleeve may comprise a screw thread or a component of a swage lock or of a bayonet fit or of a friction fit for attaching the front end to the back end. The back end would be required to have a correspondent part. This provides a means to ensure fitment of the correct front end. This may help to ensure that the intended drug is used with the reusable engine back end.

At least one barb may be arranged in the syringe cover for engaging the locking sleeve in a manner to allow the syringe cover to be re-attached over the outer sleeve when the transfer sleeve is in the needle safe position and to prevent removal of the syringe cover when the barb is engaged to the locking sleeve. In the initial position the locking sleeve and transfer sleeve are less advanced in the proximal direction than in the needle safe position thus preventing the barb from engaging and allowing removal of the syringe cover. This feature also improves post injection needle safety.

At least one snap feature may be arranged for preventing translation of the transfer sleeve in the distal direction from the needle safe position. The user can therefore not push the transfer sleeve back for needle access after removal of the front end from the injection site.

The syringe may be arranged in the front end in a manner to prevent its removal after injection. Such an arrangement may be referred to as a packaged syringe which has to be entirely disposed of after use. This embodiment ensures that contact with the user, in an area immediately surrounding the injection site, is limited to the single-use packaged syringe. This may help to prevent cross contamination between consecutive injections and may eliminate the requirement for cleaning re-usable elements. Re-use of an emptied syringe is prevented. This reduces the chance of the user being subjected to the health risks associated.

In another embodiment the syringe may be replaceably arranged in the front end.

The re-usable back end may comprise a conventional spring, a gas spring, an electric motor or another drive means for advancing the syringe and the stopper.

The front end may preferably be used for subcutaneous or intra-muscular injection, particularly for delivering one of an analgetic, an anticoagulant, insulin, an insulin derivate, heparin, Lovenox, a vaccine, a growth hormone, a peptide hormone, a protein, antibodies and complex carbohydrates.

Further scope of applicability of the present invention will become apparent from the detailed description given hereinafter. However, it should be understood that the detailed description and specific examples, while indicating preferred embodiments of the invention, are given by way of illustration only, since various changes and modifications within the spirit and scope of the invention will become apparent to those skilled in the art from this detailed description.

### Brief Description of the Drawings

The present invention will become more fully understood from the detailed description given hereinbelow and the accompanying drawings which are given by way of illustration only, and thus, are not limitive of the present invention, and wherein:
- Figure 1: are two longitudinal sections of a front end as delivered prior to being attached to a re-usable backend,
- Figure 2: is an isometric view of internal components of the front end,
- Figure 3: are two longitudinal sections of the front end with a syringe cover removed,
- Figure 4: are two longitudinal sections of the front end during needle insertion into an injection site,
- Figure 5: are two longitudinal sections of the front end being withdrawn from the injection site, and
- Figure 6: are two longitudinal sections of the front end after use during replacement of the syringe cover over an outer sleeve.

Corresponding parts are marked with the same reference symbols in all figures.

### Detailed Description of Preferred Embodiments

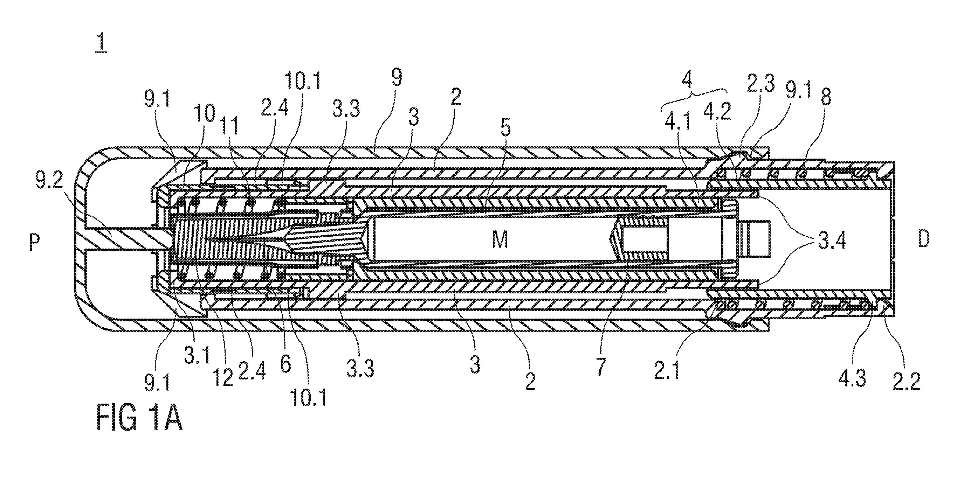

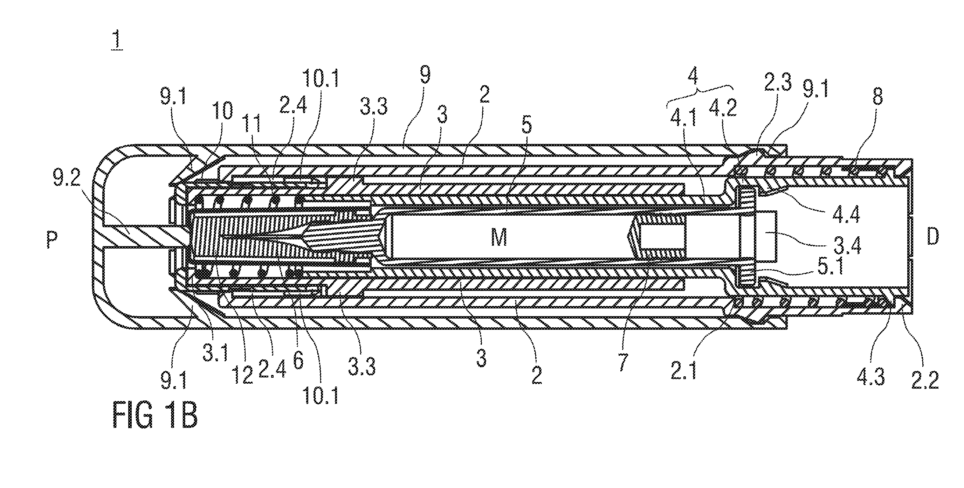

Figure 1 shows two longitudinal sections in different section planes of a front end 1 for an auto-injector, the different section planes approximately 90° rotated to each other. The front end 1 is shown in a state as delivered prior to being attached to a re-usable backend (not illustrated). The front end 1 and the reusable backend may form an auto-injector for delivering a liquid medicament M.

The front end 1 comprises an elongate outer sleeve 2. Inside the outer sleeve 2 a transfer sleeve 3 is arranged in a manner to be translatable in an axial direction. A proximal portion 4.1 of a syringe support sleeve 4 is telescoped within the transfer sleeve 3 while a distal portion 4.2 of the syringe support sleeve 4 having a greater diameter than the proximal portion 4.1 is telescoped within the outer sleeve 2. The proximal portion 4.1 is arranged to hold a syringe 5, e.g. a Hypak syringe, and support it at a proximal end so as to prevent translation of the syringe 5 in a proximal direction P relative to the syringe support sleeve 4. A finger flange 5.1 at the distal end of syringe 5 is distally held by internal clips 4.4 in the distal portion 4.2 of the syringe support sleeve 4. A hollow injection needle 6 is arranged proximally on the syringe 5 and in fluid communication with a cavity in the syringe containing the medicament M. A stopper 7 is arranged for sealing the syringe 5 distally and for displacing the liquid medicament M through the needle 6. A syringe spring 8 is arranged between a shoulder 2.1 in the outer sleeve 2 and a first rib 4.3 on the distal portion 4.2 of the syringe support sleeve 4 in a manner to bias the syringe support sleeve 4 in the distal direction D relative to the outer sleeve 2. A second rib 2.2 in the outer sleeve 2 is arranged to limit travel of the syringe support sleeve 4 by the first rib 4.3 distally abutting against the second rib 2.2.

A syringe cover 9 is initially positioned over a substantial length of the outer sleeve 2 and clipped to the outer sleeve 2 by an internal circumferential notch 9.1 in the syringe cover 9 engaged to an external circumferential third rib 2.3 on the outer sleeve 2, wherein the syringe cover 9 is arranged to prevent access to the internals of the front end 1.

A locking sleeve 10 is arranged over the transfer sleeve 3. The locking sleeve 10 has an orifice for allowing the needle 6 to pass through. A transfer spring 11 is arranged between the proximal end of the syringe support sleeve 4 and an internal proximal rib 3.1 in the transfer sleeve 3 so as to bias the transfer sleeve 3 in the proximal direction P relative to the syringe support sleeve 4 in an initial position.

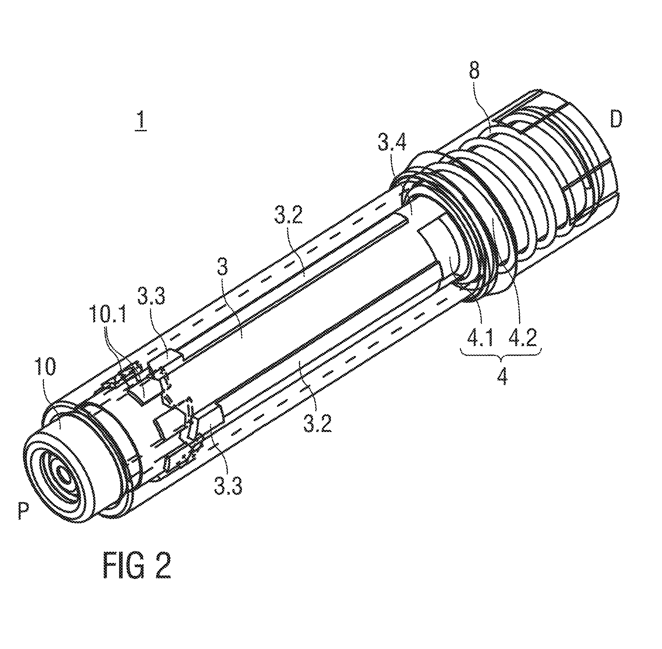

Figure 2 illustrates that the locking sleeve 10 exhibits an external circumferential shouldered castellation with a number of fourth ribs 10.1. The transfer sleeve 3 exhibits longitudinal ridges 3.2 proximally ending in ramped protrusions 3.3 with their ramps facing in the proximal direction P. Initially, the locking sleeve 10 is rotationally constrained by engagement of the fourth ribs 10.1 in corresponding first longitudinal grooves 2.4 within the outer sleeve 2. The fourth ribs 10.1, the longitudinal ridges 3.2, the ramped protrusions 3.3 and the first longitudinal grooves 2.4 are used to replicate a 'ballpoint pen' extension/retraction mechanism. The 'ballpoint pen' extension/retraction mechanism may also be achieved without the longitudinal ridges 3.2 in the transfer sleeve 3.

A sequence of operation is as follows:

A user attaches the front end 1 to the re-usable backend of the auto-injector.

This could be achieved with a screw thread, swage lock, bayonet, or friction fit.

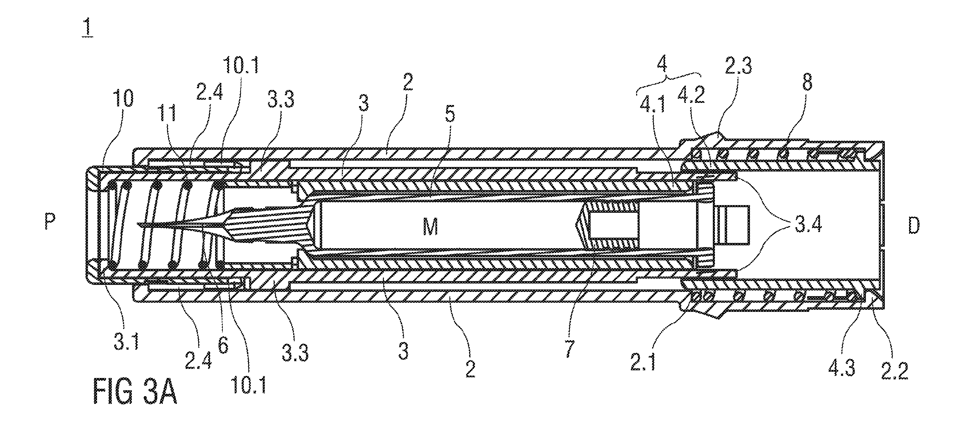

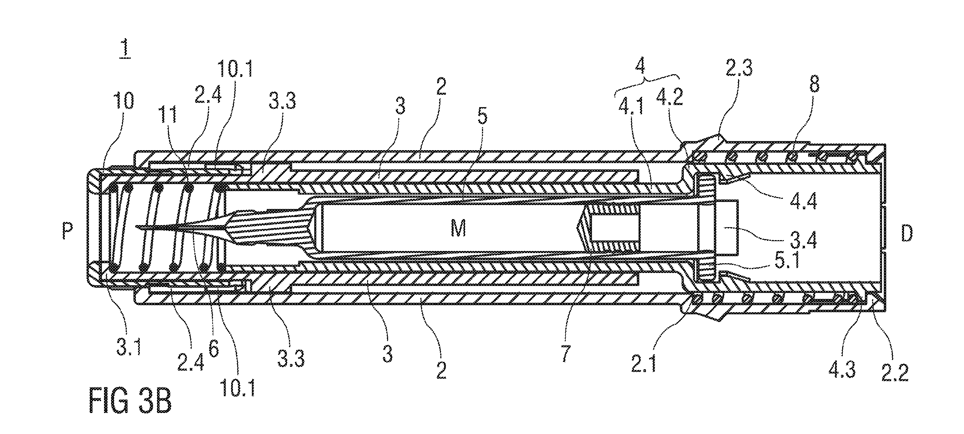

The user removes the syringe cover 9 from the outer sleeve 2 by pulling the syringe cover 9 in the proximal direction P thereby forcing a distal end of the syringe cover 9 to resiliently deflect outwards over the third rib 2.3 which is facilitated by ramps on both the third rib 2.3 and the circumferential notch 9.1 (see figures 3a and 3b). To remove the syringe cover 9, the user must apply sufficient force to deform the syringe cover 9 over the third ribs 2.3. As the syringe cover 9 is removed it engages a protective needle sheath 12 arranged over the needle 6 in a manner to remove the protective needle sheath 12. For this purpose a lug 9.2 is arranged in the syringe cover 9 extending distally from a proximal end face of the syringe cover 9. A distal end of the lug 9.2 is connected to the protective needle sheath 12.

The user positions the proximal end P of the front end 1 against the injection site, e.g. a patient's skin. As the proximal end P contacts the skin, the locking sleeve 10 is translated up inside the outer sleeve 2 against the force of the transfer spring 11.

With axial translation of the locking sleeve 10 and transfer sleeve 3 into a depressed position, the fourth ribs 10.1 are distally translated out of the first grooves 2.4 in the outer sleeve 2 and the transfer sleeve 3 and locking sleeve 10 are now free to rotate about the longitudinal axis of the transfer sleeve 3. As the locking sleeve 10 moves axially, further, into the outer sleeve 2 in the distal direction D, the fourth ribs 10.1 of the locking sleeve 10 come into contact with ramps on the outer sleeve 2 and further axial motion causes the locking sleeve 10 to rotate by a defined angle. In parallel, the axial motion of the locking sleeve is coupled to the transfer sleeve

The re-usable engine undertakes an injection cycle. The front end 1 has some functionality to assist in this process: The transfer sleeve 3 comprises distal extensions 3.4 for indicating to the re-usable engine that the front end 1 is in contact with the skin. The syringe spring 8 can be used to withdraw the needle from the skin.

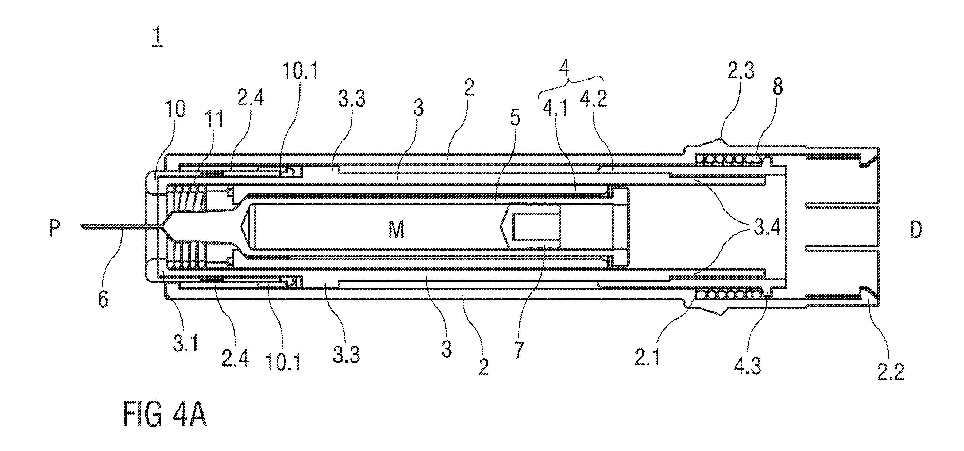

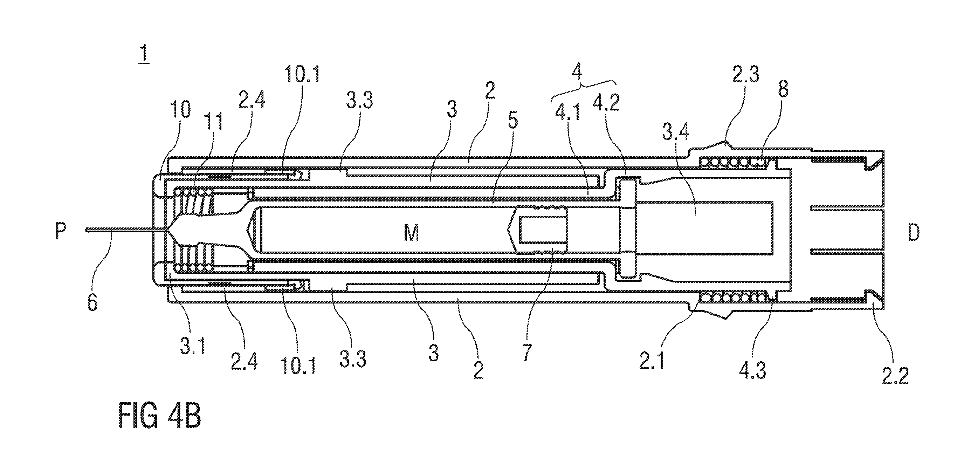

A plunger (not illustrated) on the reusable engine is arranged to push on the stopper 7. The force required to advance the syringe 5 in the proximal direction P thereby compressing the transfer spring 11 and to insert the needle 6 into the injection site is configures to be less than a force required to displace the medicament M through the injection needle 6. Hence, by pushing on the stopper 7 the syringe 5 is advanced in the proximal direction P for needle insertion (see figures 4a and 4b). A needle insertion depth is defined by the length of the completely compressed syringe spring 8 between the shoulder 2.1 and the first rib 4.3 and by the length of the completely compressed transfer spring 11 between the proximal rib 3.1 and the proximal end of the syringe support sleeve 4.

The maximum needle insertion depth could also be controlled by contact between the syringe support sleeve 4 and the outer sleeve 2.

When the injection depth has been reached continued translation of the plunger advances the stopper 7 with in the syringe 5 in the proximal direction P displacing the medicament M through the injection needle 6 into the injection site.

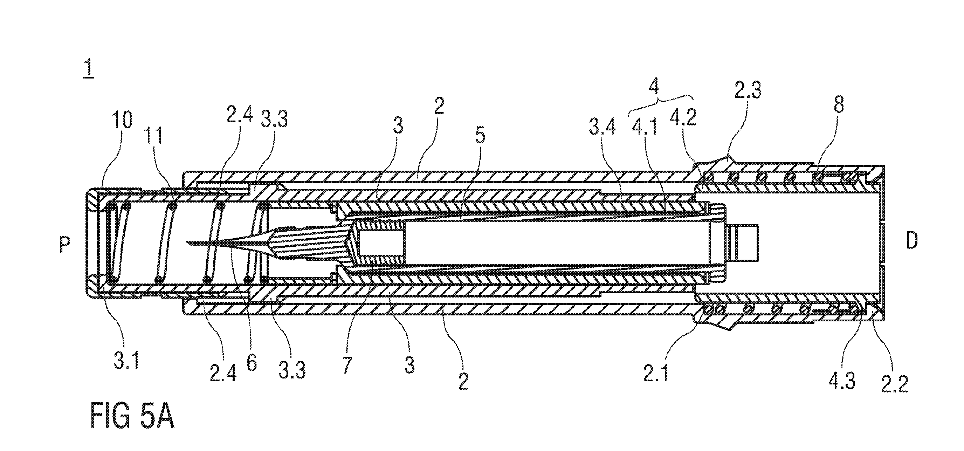

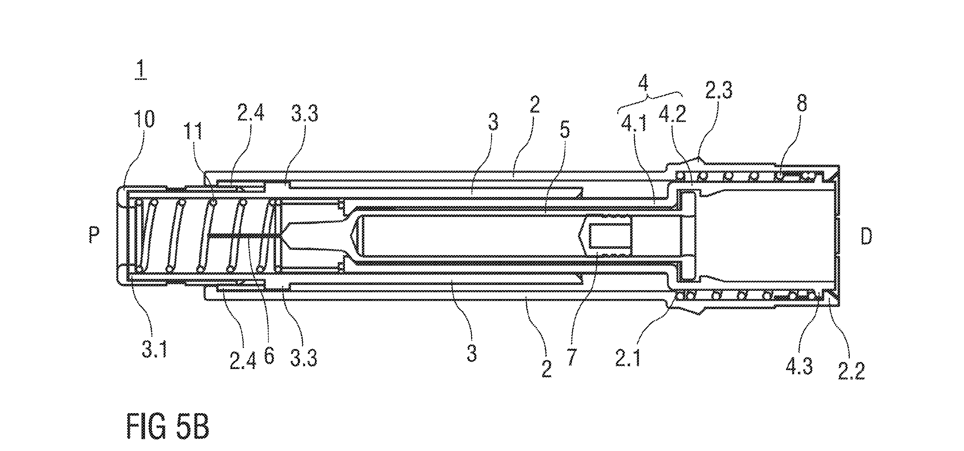

Once the injection is complete the user removes the auto-injector from the injection site. In so doing, the force compressing the transfer spring 11 is removed and the transfer sleeve 3 extends out of the proximal end of the front end 1 forced by the expanding transfer spring 11 into a needle safe position proximally beyond the initial position. This motion is coupled to the locking sleeve 10. Having rotated from its initial position, the fourth ribs 10.1 on the locking sleeve 10 engage in a different, lengthened second longitudinal groove within the outer sleeve 2 and further rotation of the locking sleeve 10 is prevented. The locking sleeve extends 10 until its fourth ribs 10.1 bottom out in the second longitudinal grooves within the outer sleeve 2. Because the second longitudinal grooves are longer, the locking sleeve 10 extends further than its pre-injection position (see figures 5a and 5b). The locking sleeve 10 is locked in position by snap features (not illustrated) on the transfer sleeve 3 engaging with the outer sleeve 2.

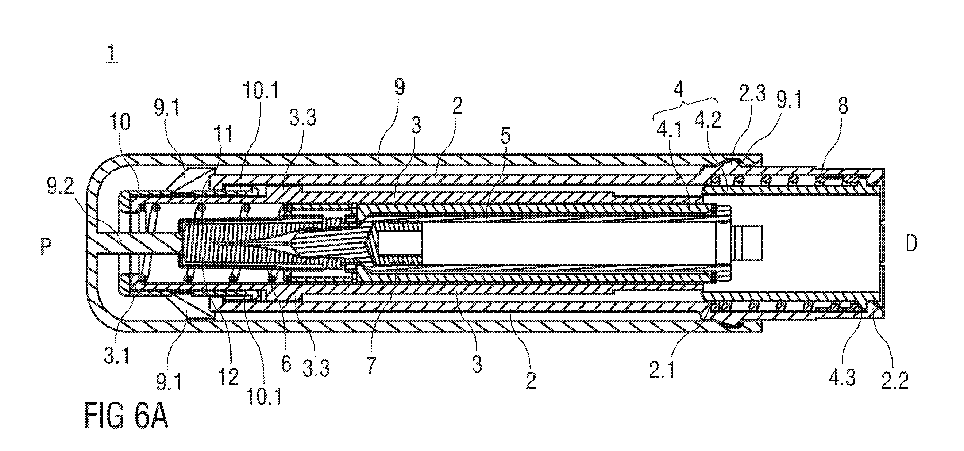

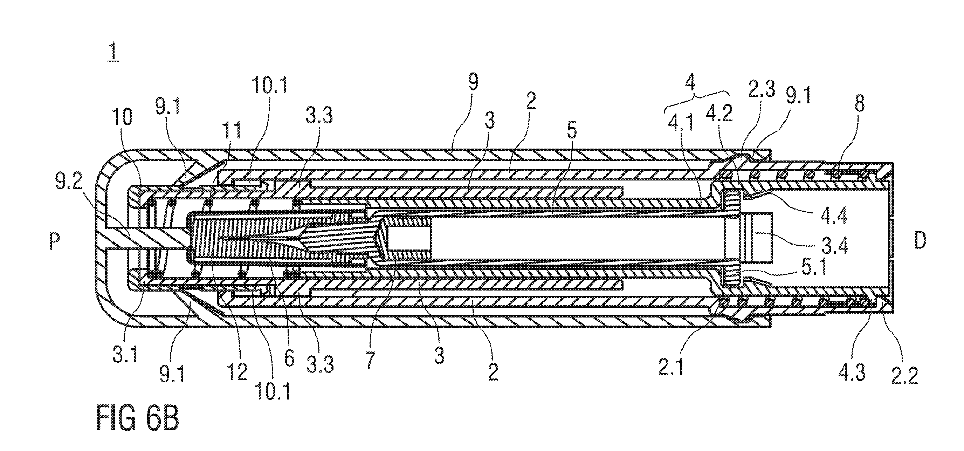

The user then replaces the syringe cover 9 over the outer sleeve 2. Because the locking sleeve 10 extends further from the proximal end P of the outer sleeve 2 than prior to use, barbs 9.1 within the syringe cover 9 engage on the outer diameter of the locking sleeve 10. This prevents the syringe cover 9 from being removed once the front end 1 has been used (see figures 6a and 6b).

The front end 1 is arranged as a packaged syringe 1 in the sense of a disposable device which is intended to be used once and to be disposed of after use. The front end 1 could also be arranged as a re-usable device, wherein the syringe 5 would be replaceably arranged in the front end 1 in such a manner that all components except the syringe 5 could be re-used.

The sense of rotation of the rotating components was illustrated by way of example. The front end 1 may easily be designed to have these components rotate in the opposite sense. This would require adequate modification of the ramped protrusions 3.3.

The front end 1 may preferably be used for subcutaneous or intra-muscular injection, particularly for delivering one of an analgetic, an anticoagulant, insulin, an insulin derivate, heparin, Lovenox, a vaccine, a growth hormone, a peptide hormone, a protein, antibodies and complex carbohydrates.

### List of References

- 1: front end
- 2: outer sleeve
- 2.1: shoulder
- 2.2: second rib
- 2.3: third rib
- 2.4: longitudinal groove
- 3: transfer sleeve
- 3.1: proximal rib
- 3.2: longitudinal ridge
- 3.3: ramped protrusion
- 3.4: distal extension
- 4: syringe support sleeve
- 4.1: proximal portion
- 4.2: distal portion
- 4.3: first rib
- 4.4: internal clips
- 5: syringe
- 5.1: finger flange
- 6: injection needle
- 7: stopper
- 8: syringe spring
- 9: syringe cover
- 9.1: barb
- 9.2: lug
- 10: locking sleeve
- 10.1: fourth rib
- 11: transfer spring
- 12: protective needle sheath
- D: distal direction
- M: medicament
- P: proximal direction

## Claims

1. Front end (1) for an auto-injector, the front end (1) comprising a syringe (5) with an injection needle (6) and a stopper (7), further comprising an outer sleeve (2) arranged to be attached to an auto-injector back end comprising a drive means for advancing the syringe (5) and needle (6) in a proximal direction (P) for needle insertion and for advancing the stopper (7) within the syringe (5) for injection, wherein the syringe (5) is slidably arranged within a transfer sleeve (3) which is slidably arranged in the outer sleeve (2), wherein the transfer sleeve (3) is biased in the proximal direction (P) against the syringe (5), wherein a locking mechanism is arranged for limiting a proximal extension of the transfer sleeve (3) from the outer sleeve (2) to an initial position in an initial state, wherein the locking mechanism is arranged to allow the transfer sleeve (3) to be extended into a needle safe position covering the advanced needle (6) beyond the initial position after having been translated in the distal direction (D) into a depressed position.

2. Front end (1) according to claim 1, **characterized in that** the locking mechanism comprises a locking sleeve (10) arranged over the transfer sleeve (3) and coupled to the transfer sleeve (3) for joint translation, wherein the locking sleeve (10) exhibits at least one fourth rib (10.1), wherein in the initial position the fourth ribs (10.1) are engaged in respective first longitudinal grooves within the outer casing (2) preventing relative rotation, wherein on translation into the depressed position the fourth ribs (10.1) are moved out of the first longitudinal grooves against respective ramp features in the outer sleeve (2) or on the transfer sleeve (3) in a manner to rotate the locking sleeve (10) by a defined angle thereby aligning the fourth rib (10.1) with a second longitudinal groove extending further in the proximal direction (P) than the first longitudinal groove thus allowing translation into the depressed position.

3. Front end (1) according to one of the claims 1 or 2, **characterized in that** a syringe support sleeve (4) is telescoped within the transfer sleeve (3), wherein the syringe support sleeve (3) is arranged to hold the syringe (5) and support it at its proximal end.

4. Front end (1) according to claim 3, **characterized in that** the syringe support sleeve (4) is biased in the distal direction (D) by a syringe spring (8) towards an initial position, wherein the syringe support sleeve (4) is arranged to be translated proximally into an insertion position for needle insertion and to be re-translated into the initial position by the syringe spring (8) for retracting the needle (6) from the injection site.

5. Front end (1) according to one of the preceding claims, **characterized in that** a syringe cover (9) is provided arranged to be removably positioned over a substantial length of the outer sleeve (2) and clipped to the outer sleeve (2), wherein the syringe cover (9) is arranged to prevent access to the internals of the front end (1) when attached to the outer sleeve (2).

6. Front end (1) according to claim 5, **characterized in that** the syringe cover (9) is arranged to engage a protective needle sheath (12) arrangeable over the needle (6) in a manner to remove the protective needle sheath (12) on removal of the syringe cover (9).

7. Front end (1) according to one of the preceding claims, **characterized in that** the transfer sleeve (3) comprises at least one distal extension (3.4) for engaging a sensor in the re-usable backend to indicate that the transfer sleeve (3) is in the depressed position.

8. Front end (1) according to one of the claims 3 to 7, **characterized in that** at least one internal clip (4.4) is arranged in the syringe support sleeve (4) for engaging a finger flange (5.1) of the syringe (5) in a manner to prevent translation of the syringe (5) in the distal direction (D).

9. Front end (1) according to one of the claims 3 to 8, **characterized in that** a transfer spring (11) is arranged between the transfer sleeve (3) and the syringe support sleeve (4) for biasing them against each other.

10. Front end (1) according to one of the preceding claims, **characterized in that** the outer sleeve (2) comprises a screw thread or a component of a swage lock or of a bayonet fit or of a friction fit.

11. Front end (1) according to one of the preceding claims, **characterized in that** the syringe (5) is replaceably arranged in the front end (1).
